# EUROPEAN PATENT APPLICATION

(11) **EP 4 494 677 A1**
(43) Date of publication of application: **22.01.2025**
(21) Application number: 23186182.4
(22) Date of filing: 18.07.2023
(51) Int. Cl.: A61M 5/32, A61M 5/42, A61M 5/46

(54) **METHOD AND SYSTEM FOR CONTROLLING MATERIAL INJECTIONS**

(71) Applicant: Agyless Medical Ltd, 4922374 Petach Tikva (IL)
(72) Inventor: BRODSKY, LEA, 4922374 41 Moshe Sneh Street, Petach Tikva (IL)
(74) Representative: Lapienis, Juozas

(57) **Abstract**

A system and method for injecting materials to a patient and for monitoring and controlling the injection process in real-time that includes a syringe, a needle, a positioning element to stabilize the needle to the skin, a force sensor to measure resistance force exerted on the needle during penetration, and a controller that receive from the sensor data measurement of the resistance force. The controller detects in real time the location of the tip along the tissue layer, detects a contact with blood vessels, and enables to inject the materials in the upper, middle or lower part of the specific tissue layer, depending on the needed treatment, and to prevent penetration of the needle into blood vessels.

## Description

### FIELD OF THE INVENTION

The present invention refers to a system for injecting filler materials or other kinds of materials into a patient and monitoring and controlling the injection process in real-time.

### BACKGROUND OF THE INVENTION

PCT/IL2016/050210 of the present inventor discloses a method and system for detecting blood vessels during injections. The present invention discloses an invention for detecting and locating the place of the injection along the dermis (or other skin tissues of the patient) and better ways for detecting blood vessels. Many aesthetic and medical procedures involve the injection of filler materials, and it is important to make the injection in the proper location along the dermis or other skin tissue layer depending on the specific injected material and condition of the patient and also to avoid the injection of the substances into blood vessels, and often it is important to inject the filler materials specifically into the upper part, or the middle part or the lower part of the dermis or other skin layer of the patient. The present invention discloses a system for achieving these goals.

### SUMMARY OF THE INVENTION

The present invention discloses a method and a system (100) for injecting materials (200) to a patient (300) (in the drawings only the skin of the patient is shown) and for monitoring and controlling the injection process in real-time. The method and the system comprise the followings steps and elements:
(a) A syringe (8) that can be a standard one;
(b) A needle (7) that is connected or designed to be connected to the syringe;
(c) A positioning element (1) that is designed to stabilize the needle in relation to the skin (6) of the patient.
(d) A force sensor (9) that is designed to measure the resistance force exerted on the needle during penetration of the needle within the tissues of the patient, and
(e) A controller (21) that is designed to receive from the force sensor data measurement on the resistance force.

The controller is designed to detect in real-time the penetration of the tip of the needle into the dermis layer (20) of the patient, based on the data measurement on the resistance force, or into other kind of tissue layers such as the epidermis (19), subcutaneous tissue (26) or muscle (25) and to detect blood vessels (22).

The controller is designed to detect in real-time the location (400) of the tip of the needle along the dermis layer or along other kinds of tissue layers. The controller is designed to do that based on the data measurement of the resistance force and/or time and distance of movement of the needle along the dermis or along other kinds of tissue layer and based on analysis of the controller of other parameters.

The resistance force of the tissues on the tip of the needle and on the needle itself during the penetration of the needle into the patient's tissues is used by the controller as an indication of the location (position) of the needle within the tissues, that is, what type of tissue the needle passes through and what is the depth of the tip position in that tissue in real-time. For example, when the needle penetrates the skin (epidermis), a high resistance is detected for a very short period of time, and then there is a more or less constant resistance, which is an indication that the needle is moving deep into the dermis, and when a sharp and small change in resistance is detected, this used by the controller as an indication that the tip of the needle has encountered in a blood vessel, and in order not to damage it the controller sends an immediate alert for the therapist to stop the insertion operation (if it is a manual insertion) or an automatic command to stop the motor that performs the insertion operation of the needle or when the treatment need to inject the material into the blood vessel then to penetrate it and inject into it.

The controller, as its name indicates, may include software (which can be simple or complex) that is designed to analyze the strength of the resistance at any given moment, to compare it to a previous strength of resistance (identifying changes in the strength of the resistance), to identify the magnitude of the change, and it is possible to also compare it against accepted and standard strength of changes, and issue an output result where the tip of the needle is located (i.e.: in which tissue layer and the depth and in which part of the tissue layer), and the software can also include an algorithm that will help it ignore background noise (for example, negligible changes that could indicate the needle encountering sebaceous glands or sweat glands).

The controller is designed to detect in real time contact of the tip of the needle with a blood vessel (22), based on the data measurement on the resistance force received in real-time from the force sensor as explained above. In addition to that, when the controller detects another change in the resistance force, it is used by the controller as an indication that the tip moved out of the dermis (or another kind of tissue) and arrived at the muscle (or another kind of tissue). This information, as itself and also possibly together with a timer, can create a sort of 'map' of the dermis and the other kind of tissues and enable the controller to detect when the tip is in the upper part (20U) of the dermis, when in the middle part (20M) and when in the lower part (20L) and in many cases it is important to inject the materials in a specific part of the dermis depending on the situation of the patient, for example, how deep is the wrinkle that we want to straighten.

These characteristics of the controller enable the therapist to inject the materials (such as filler materials) in the upper part, in the middle part, or in the lower part of the dermis layer or other skin tissue layer, depending on the specific treatment and also enable to stop the movement of the needle for preventing penetration into blood vessels.

In general, we can say that there is a need for a system and a method for detecting in real-time blood vessels during the injection and for detecting in which kind of tissue the tip and in which depth. Sometimes there is a need to prevent penetration into blood vessels (to make sure also not to inject the materials into them), and sometimes there is a need to find blood vessels and inject the materials into them.

The system and the method use at least one force sensor reactive to the needle or cannula insertion force linked to a controller, which reacts to the measured force profile In embodiments of the present invention there is provided a method, system, and device for detection of blood vessel penetration or prevention of blood vessel penetration during needle insertion either for preventing injection of the substance within a blood vessel or for ensuring its injection within a blood vessel or for different applications where blood vessels detection or blood vessels penetration detection is essential.

The system and the method also use a positioning element that is placed on the patient's skin. The positioning element stabilizes the device in relation to the patient's skin surface and does not press skin layers at the injection site. The system and the method also use a force sensor that allows the measurement of the needle's insertion force required to overcome the patient's tissues resistance to the needle penetration and a controller, functionally coupled and/or linked to the force sensor, which performs force measurement analysis for blood vessel penetration prevention, and/or for needle's condition detection and/or for blood vessel penetration detection.

When a blood vessel is detected, the controller may optionally prevent the injection, and the penetration, or it may enable the injection into the blood vessel, according to the need.

The combination of the positioning element that stabilizes the device and the needle in relation to the patient's skin surface and does not press the patient's skin layers in the injection site and the force sensor measurement analysis allow a precise detection of contact with the blood vessel and precise detection of blood vessel penetration.

The positioning element allows the practitioner to position the device on the patient's skin, stabilizing the device in relation to the patient's skin surface during the injection process.

The positioning element is designed in such a manner that it may press the patient's skin layers in an area nearby or surrounding the injection site (the injection point), but not in the injection site itself or too close to it.

The positioning element may be designed in such a manner that it does not press the patient's skin layers in the treated area, allowing the practitioner to see the injection site and the treated area clearly. The positioning element may be designed in such manner that it may be placed in contact with the patient's skin, stabilizing the needle in parallel to the patient's skin surface, or perpendicular to the skin surface or at a desired angle relative to the patient's skin surface.

In one embodiment of this invention, the device's purpose is to inject one of the currently available dermal fillers for wrinkle treatment avoiding penetration into the blood vessels under the patient's skin surface. In another embodiment of this invention, the device's purpose is to inject other liquids or gels avoiding penetration into the blood vessels under the patient's skin surface.

In one embodiment of this invention, the device's purpose is to inject one of the currently available dermal fillers for wrinkle treatment, avoiding injecting into the blood vessels under the patient's skin surface. In another embodiment of this invention, the device's purpose is to inject other liquids or gels, avoiding injecting into the blood vessels under the patient's skin surface.

In one embodiment of this invention, the device's purpose is to inject a substance into a blood vessel, avoiding its injection outside the blood vessel.

In one embodiment of this invention, the device's purpose is to inject one of the currently available dermal fillers for wrinkle treatment while detecting the needle's condition during its penetration under the patient's skin surface. In another embodiment of this invention, the device's purpose is to inject other liquids or gels while detecting the needle's condition during its penetration under the patient's skin surface.

In one embodiment of this invention, the device includes a disposable transparent positioning element that is in contact with the patient's skin during the injection process. The positioning element has a hole in its tip to allow the needle to move through a hole and to penetrate the patient's skin during the injection. As a result of the location of the hole (in the tip of the positioning element) and size, the patient's skin layers are not pressed in the needle penetration point, allowing precise blood vessel detection or blood vessel penetration detection.

In another embodiment of this invention, the device includes a positioning element that is in contact with the patient's skin during the injection process. Positioning element may be disposable or not, may be transparent or not, and may be made of any suitable material and may have any suitable shape, as long as it is able to stabilize the device in relation to the patient's skin surface.

In one embodiment of this invention, a needle is attached to the tip of a syringe. In another embodiment of this invention, a flexible tube links the syringe, and the needle, and the needle is displaced independently of the syringe or container.

In one embodiment of this invention, a force sensor may be placed on top of the syringe barrel. In this embodiment, the syringe barrel is pushed by an actuator during syringe displacement. In another embodiment of this invention, aforce sensor may be placed on the top of the syringe's plunger, or it may be placed in any other suitable location that allows measuring the needle's insertion force required to overcome the patient's tissues' resistance to needle penetration.

In one embodiment of this invention a force sensor is connected to an electronic circuit, which functions as a device controller. The force sensor's output signal is measured, read, and analyzed by the device controller. According to the force sensor signal analysis, a controller determines whether the needle's tip has touched a blood vessel wall or whether a needle has penetrated a blood vessel.

The controller electronic circuit controls an actuator, which is responsible for syringe/needle displacement, and it can optionally stop the syringe's/needle's displacement in case of contact with blood vessel detection or in case of blood vessel penetration detection. The controller electronic circuit controls an actuator, which is responsible for the syringe's plunger displacement, and in case a blood vessel has been detected or a blood vessel penetration event has been detected, it may prevent plunger displacement, thus preventing the injection of liquid or gel, or may allow the plunger displacement, thus injecting the substance into the blood vessel as intended, according to the application.

In one embodiment of this invention, the force sensor is connected to an electronic circuit, which functions as a device controller. The force sensor's output signal is measured, read, and analyzed by the device controller. According to the force sensor signal analysis, a controller may determine whether the needle is not sharp, bent, or defective and may inform the practitioner accordingly.

In one embodiment of this invention, an actuator may be an electrical motor. In another embodiment of this invention, an actuator may be a pneumatic, hydraulic or any other suitable actuator able to push and displace the syringe or the needle.

In one embodiment of this invention, the syringe displacement motor may be an electrical motor, which, using a mechanical transmission, moves the syringe in a linear path.

In one embodiment of this invention, a motor may be a step motor. In another embodiment of this invention linear motor may be a D.C. brush motor, a D.C. brushless motor, an AC motor, or any kind of electric motor with an added motion, rotation, or position detector or sensor for either directly or indirectly measuring needle's movement and for determination of needle's position. Motion, rotation or position detector may be mechanical, electrical, optical or of any suitable kind.

In one embodiment of this invention, the device may activate an audible alarm in case contact with a blood vessel event has been detected. In another embodiment of this invention, the device may activate an optical, vibratory or any kind of suitable alarm in case contact with a blood vessel event has been detected, in order to inform the practitioner of such event.

In one embodiment of this invention, the device may activate an audible alarm in case a blood vessel penetration event has been detected. In another embodiment of this invention, the device may activate an optical, vibratory or any kind of suitable alarm in case a blood vessel penetration event has been detected, in order to inform the practitioner of such event.

In another embodiment of this invention, the device may activate an optical, vibratory or any kind of suitable alarm in case a blood vessel penetration event has not been detected prior to injecting the substance, in order to inform the practitioner of such event.

In one embodiment of this invention the needle is inserted in parallel to the patient's skin surface. In another embodiment of this invention the needle is inserted perpendicularly to the patient's skin surface, or it may be inserted at any suitable angle in relation to the patient's skin.

Unless otherwise defined the various embodiment of the present invention may be provided to an end user in a plurality of formats, platforms, and may be outputted to at least one of a computer readable memory, a computer display device, a printout, a computer on a network or a user.

Unless otherwise defined, all technical and scientific terms used herein have the same meaning as commonly understood by one of ordinary skill in the art to which this invention belongs. The materials, methods, and examples provided herein are illustrative only and not intended to be limiting.

Implementation of the method and system of the present invention involves performing or completing certain selected tasks or steps manually, automatically, or a combination thereof.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a schematic illustrative diagram of an exemplary embodiment according to the present invention, showing the tip of a disposable positioning element and needle;
FIG. 2 is a schematic illustrative diagram of an exemplary embodiment according to the present invention, shows the tip of the disposable positioning element and needle (transparent);
FIG. 3A is a schematic illustrative diagram of an exemplary embodiment according to the present invention, shows a vertical central cutout of the disposable positioning element;
FIG. 3B illustrates the tissues layers;
FIG. 4 is a schematic illustrative diagram of an exemplary embodiment according to the present invention, shows a vertical central cutout of the disposable positioning element placed on patient's skin, including the needle, the skin layers and a blood vessel;
FIGS 5A - 5F are a schematic illustrative embodiment according to the present invention, shows the skin, the needle and a blood vessel position during different stages of the injection process;
FIG. 6 is a schematic illustrative diagram of an exemplary embodiment according to the present invention, shows the device configuration; and
FIG. 7 is a schematic illustrative diagram of an exemplary embodiment according to the present invention, shows an oscillogram of the force sensor measurement registered by an oscilloscope during needle penetration of the skin surface, the oscillogram showing measured voltage as a function of time.

### DESCRIPTION OF THE PREFERRED EMBODIMENTS

The principles and operation of the present invention may be better understood with reference to the drawings and the accompanying description. The following figure references labels are used throughout the description to refer to similarly functioning components are used throughout the specification herein below.
- 1: disposable transparent positioning element;
- 2: disposable transparent positioning element's tip;
- 3: disposable transparent positioning element tip's center hole;
- 4: patient's skin surface;
- 5: pressed patient's skin layers;
- 6: not pressed patient's skin layers;
- 7: needle;
- 8: syringe;
- 9: force sensor;
- 10: syringe and force sensor holder;
- 11: piston of linear motor for syringe displacement ;
- 12: linear motor for syringe displacement;
- 13: syringe plunger;
- 14: piston of linear motor for pushing the plunger;
- 15: linear motor for pushing the plunger;
- 16: device enclosure;
- 17: moment when needle tip touches the skin surface (measured by force sensor);
- 18: moment when needle tip penetrates the skin (measured by force sensor);
- 19: epidermis;
- 20: dermis;
- 21: device controller;
- 22: blood vessel;
- 23: moment when needle tip touches the blood vessel (measured by force sensor);
- 24: the moment when needle tip penetrates the blood vessel (measured by force sensor).

The present invention is described with reference to the accompanying drawings. According to this invention there is provided a method, system and device for detection of blood vessels or prevention of blood vessel penetration for safely injecting of liquids or gels under the skin surface .

The device allows prevention of blood vessel penetration, thus preventing blood vessels' injury (bruising) and/or allows detection of blood vessel penetration, thus preventing injection of liquid or gel into the blood vessel under the skin surface either for wrinkle treatment or for other purposes.

The drawings are schematic, not showing all the details such as the securing elements of the various items, for the sake of simplicity and clarity .

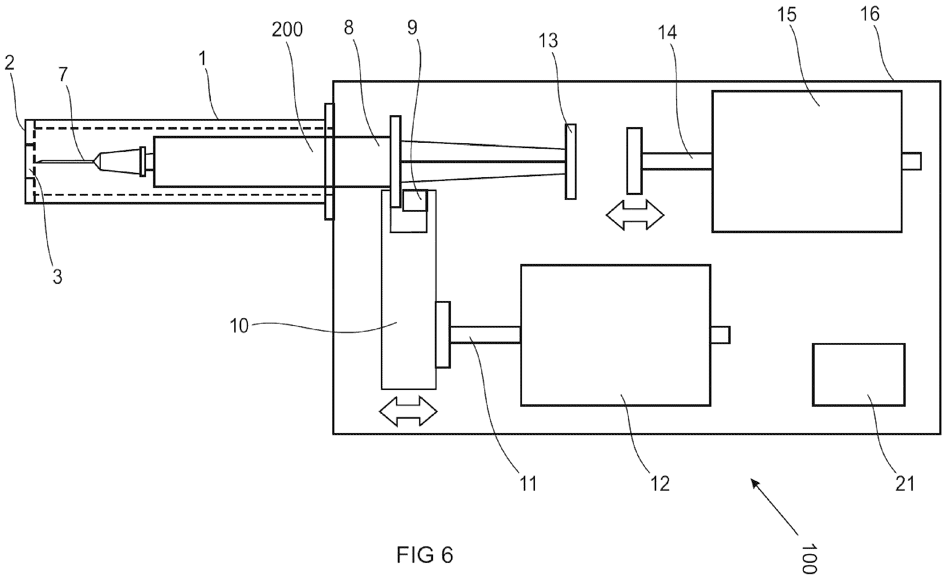

In the preferred embodiment of this invention the device enclosure 16 is designed for being hand-held by the operator, as shown in FIG. 6. The operator places the device on the patient's skin, just on top of the skin area to be treated, for instance a wrinkle. The device should be placed on the treated skin area in such manner that the disposable transparent positioning element's tip 2 is in full contact with the area to be treated. The disposable transparent positioning element tip's center hole 3 should be placed precisely on the intended injection site.

A thin needle 7 is mounted on the tip of the syringe 8. In order to perform the injection, the linear motor for syringe displacement 12 and the syringe displacement linear motor's piston 11 are activated by the device controller 21. The activation of the linear motor for syringe displacement 12 and syringe displacement linear motor's piston 11 moves the syringe 8 and the needle 7 together either forward or backwards, allowing needle's insertion into patient's skin or its extraction from the patient's skin.

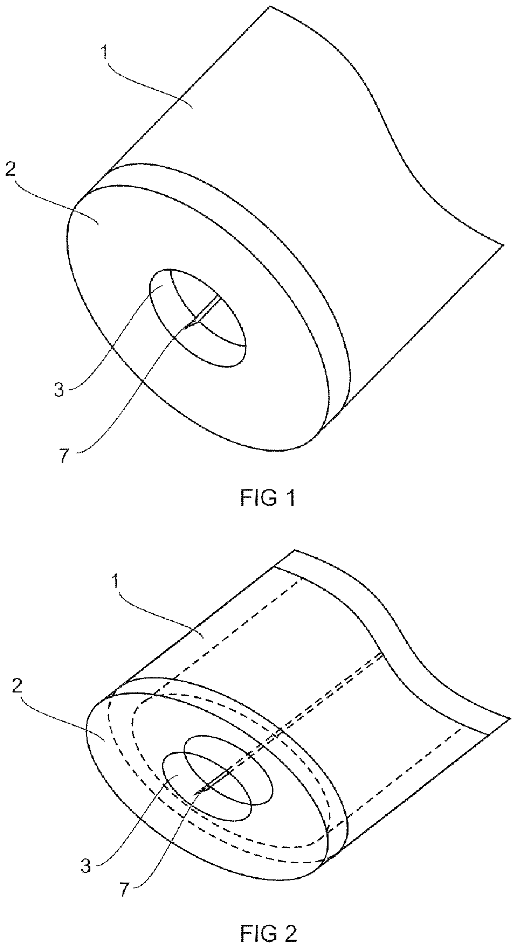

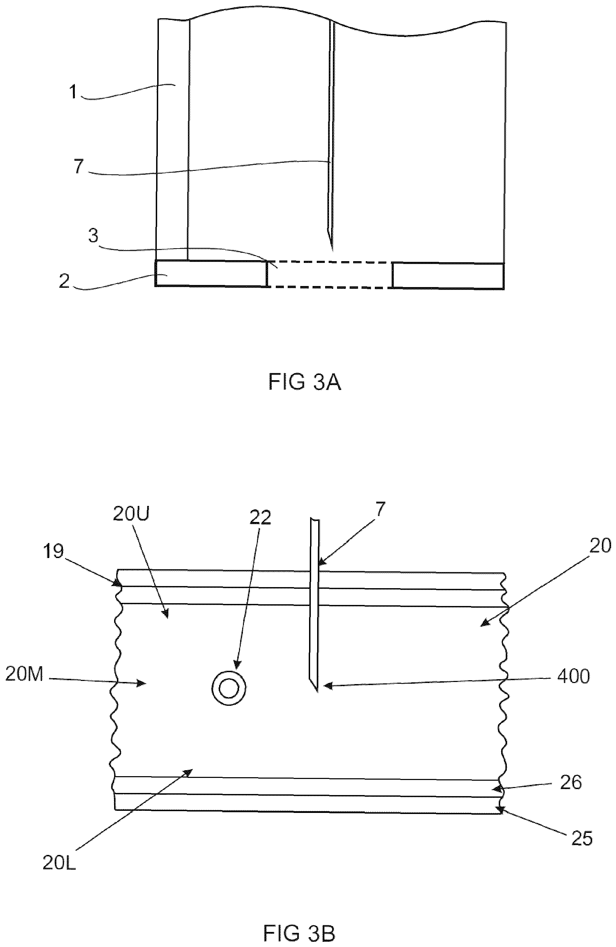

The disposable transparent positioning element 1 is shown in FIG. 1 as an opaque element, and in FIG. 2 as a transparent element. FIG.3A shows a vertical central cutout of the disposable transparent element 1. The disposable transparent element 1 houses syringe 8 with the needle 7 attached to the syringe's 8 tip. The disposable transparent positioning element 1 has a tube shape with a flat tip 2. Flat tip 2 has a round hole 3 in its center, which allows to position the device on the patient's skin surface in such a manner that the device is completely stable and the skin layers, epidermis 19 and dermis 20, are not pressed at the injection site, allowing precise measurement of the needle's insertion force required to overcome the patient's tissues resistance to the needle 7 insertion.

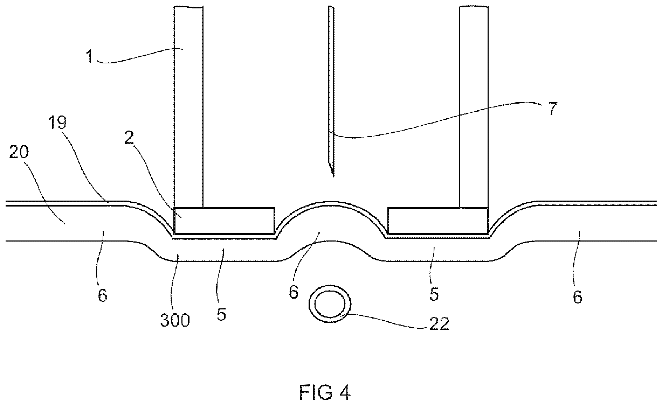

FIG. 4 depicts the skin layers of the patient and the blood vessel 22, showing the pressed skin layers 5 under the solid part of the disposable transparent positioning element's tip 2, and the not-pressed skin layers 6 in the central area of the disposable transparent positioning element's tip center hole 3. The external, thin skin layer is the epidermis 19, and under it the thicker dermis layer 20 as shown. Figure 3B illustrates the dermis and its parts.

Force sensor 9 is placed on top of the syringe 8 barrel, which is pushed by the piston 11 of the linear motor for syringe displacement 12 during syringe displacement. Detection of event when needle 7 contacts blood vessel's 22 is determined by the needle's insertion force measured by the force sensor 9 during needle 7 insertion into the patient's skin. Device controller 21 receives output signal from the force sensor 9 and, accordingly, can stop the needle 7 insertion process .

Upon detection of needle's 7 contacts with a blood vessel 22, device controller 21 immediately stops the operation of linear motor for syringe displacement 12, preventing any further insertion of the needle 7 into the blood vessel 22, thus preventing the blood vessel 22 penetration by the needle 7.

Device controller 21 may stop the operation of the linear motor for pushing the plunger 15, preventing the injection of liquid or gel adjacent to the blood vessel.

Detection of blood vessel penetration by the needle 7 is determined by the needle's 7 insertion force measured by the force sensor 9 during needle 7 insertion into the patient's skin. Device controller 21 receives output signal from the force sensor 9 and, accordingly, can stop the needle's 7 insertion process. Device controller 21 stops the operation of the linear motor for pushing the plunger 15, preventing the injection of liquid or gel into the blood vessel.

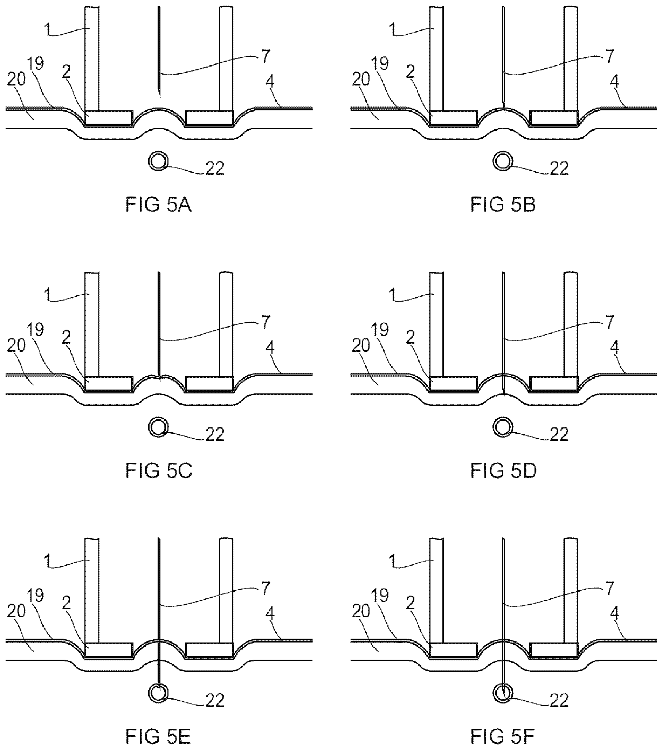

FIG. 5A-F show the disposable transparent element 1, the needle 7, the patient's skin surface 4, the skin epidermis 19, the skin dermis 20 and the blood vessel 22 in six stages FIG. 5A-F showing the progression of the needle's 7 penetration prior to the injection process, including needle's 7 contact with blood vessel 22, and also needle's 7 penetration into a blood vessel 22 .

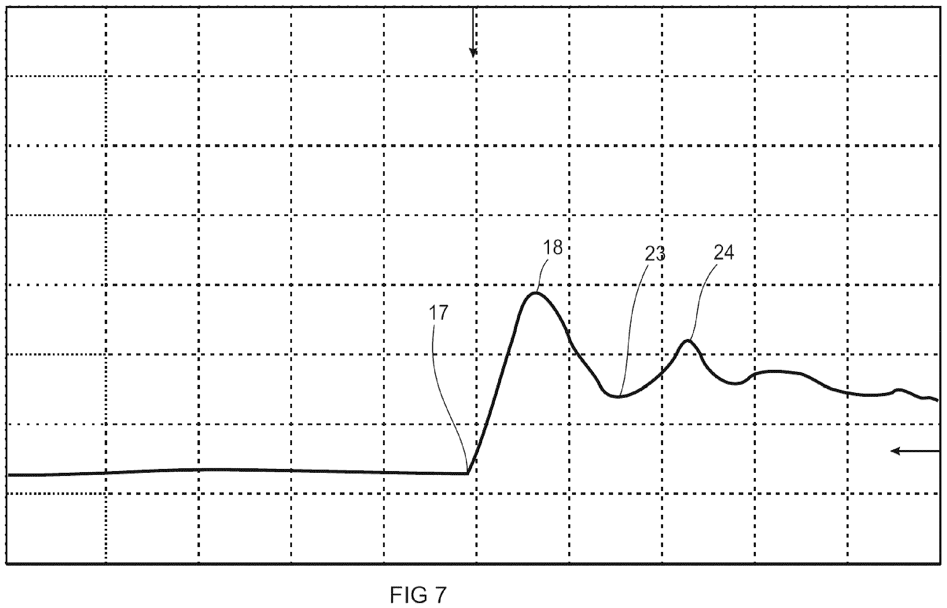

FIG. 7 shows an oscillogram of the force sensor (9) signal recorded during needle's 7 penetration into the skin and penetration into a blood vessel 22, which this method and device prevents. The vertical axis reflects the force measured by the force sensor 9 and the horizontal axis reflects the time elapsed. Since the linear motor for syringe displacement 12 operates at constant speed, the horizontal axis represents syringe's 8 and needle's 7 displacement in a linear scale.

First, the needle 7 is at a certain distance from the skin surface 4 as shown in FIG. 5A. At this stage force sensor 9 senses only the frictional force of the advancing syringe 8 and the oscillogram shows a flat line.

As the needle 7 is displaced further toward the skin surface, it touches the epidermis 19 as shown in FIG. 5B. At this stage force sensor 9 starts sensing the force required to overcome the resistance of the epidermis 19 to needle's 7 tip insertion. Accordingly, the force measurement chart shows a steep increase in the sensed force, as shown in the inflexion point 17 in FIG.7. As the needle 7 further pushes the skin layers down as depicted in FIG.5c, the force measured by the force sensor 9 increases, as shown in FIG. 7 between points 17 and 18.

The needle 7 moves further forward, until the patient's skin is pierced as shown in Fig. 5d. The patient's skin penetration by the tip of the needle 7 happens at the moment 18 in FIG. 7. At this point the needle 7 insertion force required to overcome the skin resistance measured by force sensor 9 begins to decrease.

If the force measured at point 18 is higher than it should be when operating under normal conditions, then we can conclude that the needle's 7 condition is not good, either needle 7 is not sharp, or bent, or the needle 7 is defective in some manner.

In case the force measured at the peak point 18 increases by a predetermined amount or factor after a number of injections have been carried out, the device controller 21 activates a red LED (not shown), informing the practitioner that needle 7 is not sharp.

In case the force measured at the peak point 18 is higher than a predetermined threshold, the device controller 21 activates a yellow LED (not shown), informing the practitioner that needle 7 is defective.

The needle 7 moves deeper under the skin and the needle's 7 tip touches the blood vessel 22 wall as shown in FIG. 5E, point 23 in FIG. 7. At this point the needle 7 insertion force required to overcome the tissue resistance force measured by force sensor 9 begins to increase, as shown in FIG. 7 between points 23 and 24.

In order to prevent blood vessel's 22 penetration that would happen at point 24, the needle 7 insertion is stopped by device controller 21 somewhere between points 23 and 24 as shown in FIG. 7, after it identified a raise in the force measured by force sensor 9. The syringe displacement linear motor's (12) operation is stopped by the device controller 21 when the needle's 7 tip touches and very slightly pushes the blood vessel 22 wall, thus preventing blood vessel penetration.

The device controller 21 doesn't activate then the linear motor for pushing the plunger 15, preventing the injection of liquid or gel in case a blood vessel was detected. The device controller 21 activates the linear motor for syringe displacement 12 backwards, taking the syringe 8 back inside the disposable transparent positioning element 1.

The device controller 21 activates an on-board beeper (not shown), informing the practitioner that a blood vessel has been detected by the needle 7, and that the current injection process has been stopped. In case the needle 7 has not touched a blood vessel, the syringe displacement linear motor's 12 operation is stopped by the device controller 21 when the desired penetration depth is achieved.

In order to prevent injection of liquid or gel into a blood vessel, assuming that following the stage depicted in FIG. 5E the needle 7 keeps moving forward, it penetrates the blood vessel's 22 wall, as shown in FIG. 5F. At this point the needle 7 insertion force required to overcome the blood vessel 22 resistance force measured by force sensor 9 peaks and begins to decrease, as shown in FIG. 7 point 24. The device controller 21 detects a peak 24 shown in FIG. 7, which indicates the penetration of a blood vessel 22 by the needle 7 .

The device controller 21 doesn't activate then the linear motor for pushing the plunger 15, preventing the injection of liquid or gel inside a blood vessel. The device controller 21 activates the linear motor for syringe displacement 12 backwards, taking the syringe 8 back inside the disposable transparent positioning element 1.

The device controller 21 then activates an on-board beeper (not shown), informing the practitioner that a blood vessel penetration event has occurred, and that the current injection process has been stopped.

While the invention has been described with respect to a limited number of embodiment, it is to be realized that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

Therefore, the foregoing is considered as illustrative only of the principles of the invention. Further, since numerous modifications and changes will readily occur to those skilled in the art, it is not described to limit the invention to the exact construction and operation shown and described and accordingly, all suitable modifications and equivalents may be resorted to, falling within the scope of the invention.

While the invention has been described with respect to a limited number of embodiment, it is to be realized that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the invention. Section headings are used herein to ease understanding of the specification and should not be construed as necessarily limiting.

While the invention has been described with respect to a limited number of embodiments, it will be appreciated that many variations, modifications and other applications of the invention may be made.

## Claims

1. A system for injecting materials into a patient and for monitoring and controlling the injection process in real-time, comprising:
a syringe,
a needle that is connected or designed to be connected to the syringe,
a positioning element that is designed to stabilize the needle in relation to the skin of the patient,
a force sensor that is designed to measure resistance force exerted on the needle during penetration of the needle within tissues of the patient, and
a controller that is designed to receive from the force sensor data measurement on said resistance force;
wherein the controller is designed to detect in real-time penetration of the tip of the needle into the tissue of the patient, and to identify the depth of the needle in the tissue and the kind of tissue layer that the tip of the needle is positioned, based on said data measurement on said resistance force and/or analysis made by the controller;
wherein the controller is designed to detect in real-time the location of the tip of the needle along the specific tissue layer;
wherein the controller is designed to detect in real time a contact of the tip of the needle with a blood vessel, based on the data measurement on said resistance force received in real-time from the force sensor; and
wherein enabling to inject the materials in an upper part, a middle part or a lower part of a dermis layer or in another kind of tissue layer, depending on the specific treatment, and/or enabling to stop the movement of the needle in order to prevent penetration into blood vessels or directing the tip into the blood vessel in order to inject the material into the blood vessel.

2. A method for a real-time controlling and monitoring an injection process of materials into a patient, the method comprising:
providing a syringe, a needle that is connected or designed to be connected to the syringe, a positioning element that is designed to stabilize the needle in relation to the skin of the patient, a force sensor that is designed to measure resistance force exerted on the needle during penetration of the needle within tissues of the patient, and a controller that is designed to receive from the force sensor data measurement on said resistance force;
wherein the controller is designed to detect in real-time penetration of the tip of the needle into the tissue of the patient, and to identify the depth of the needle in the tissue and the kind of tissue layer that the tip of the needle is positioned, based on said data measurement on said resistance force and/or analysis made by the controller;
wherein the controller is designed to detect in real-time the location of the tip of the needle along the specific tissue layer;
wherein the controller is designed to detect in real time a contact of the tip of the needle with a blood vessel, based on the data measurement on said resistance force received in real-time from the force sensor; and
enabling to inject the materials in an upper part, a middle part or a lower part of a dermis layer or in another kind of tissue layer, depending on the specific treatment, and/or enabling to stop the movement of the needle in order to prevent penetration into blood vessels or directing the tip into the blood vessel in order to inject the material into the blood vessel.
